# EUROPÄISCHE PATENTSCHRIFT

(11) **EP 0 620 005 B1**
(45) Veröffentlichungstag und Bekanntmachung des Hinweises auf die Patenterteilung: **01.12.1999**
(21) Anmeldenummer: 94105017.1
(22) Anmeldetag: 30.03.1994
(51) Int. Cl.: A61K 31/72

(54) **Verwendung von Hydroxyethylstärke zur Verbesserung der Mikrozirkulation**
Use of hydroxyethylstarch for the improvement of the microcirculation
Utilisation de l'hydroxyéthylamidon pour l'amélioration de la microcirculation

(30) Priorität: 03.04.1993 DE 4310974
(43) Veröffentlichungstag der Anmeldung: 19.10.1994
(73) Patentinhaber: Fresenius AG, 61350 Bad Homburg (DE)
(72) Erfinder: Weidler, Burghard, Prof. Dr., D-61191 Rosbach v.d. H. (DE); Sommermeyer, Klaus, Dr., D-61191 Rosbach v.d. H. (DE); Henning, Klaus, Dr., D-61250 Usingen (DE); Bepperling, Frank, Dr., D-61191 Rosbach v.d. H. (DE)
(74) Vertreter: Luderschmidt, Schüler & Partner GbR

(56) Entgegenhaltungen:
- EP-A- 0 402 724
- DE-A- 3 030 863
- DE-A- 4 023 789
- INFUSIONSTHERAPIE, Bd.17, Nr.2, 1990 Seiten 79 - 82 KORTSIK, C.F. ET AL 'KONJUNKTIVALER SAUERSTOFFPARTIALDRUCK, HAMORHEOLOGIE UND KREISLAUFPARAMETER BEI AKUTEM ZEREBRALEM INSULT VOR UND NACH INFUSION VON 6PROZENTIGER NIEDERMOLEKULARER HYDROXYATHYLSTARKE'
- ARZNEIMITTELFORSCHUNG, Bd.43(I), Nr.2, 1993 Seiten 99 - 105 JUNG, F. ET AL 'EINFLUSS DER MOLEKULSTRUKTUR VON HYDROXYATHYLSTARKE AUF DIE ELIMINATIONSKINETIK UND DIE FLIESSFAHIGKEIT DES BLUTES BEI PROBANDEN'
- ARZNEIMITTELFORSCHUNG, Bd.41(I), Nr.5, 1991 Seiten 494 - 498 WEIDLER, B. ET AL 'PHARMAKINETISCHE MERKMALE ALS KRITERIEN FUR DEN KLINISCHEN EINSATZ VON HYDROXYATHYLSTARKE'

## Beschreibung

Gegenstand der vorliegenden Erfindung ist die Verwendung einer Hydroxyethylstärke-Spezifikation mit einem Molekulargewicht M_{w} von 110.000 bis 150.000, einem Substitutionsgrad MS von 0,38 bis 0,5, einem Substitutionsgrad DS von 0,32 bis 0,45 und einem Verhältnis von C₂/C₆ von 8 bis 20 zur Verbesserung der Mikrozirkulation bei peripherer arterieller Durchblutungsstörung, insbesondere bei bereits bestehender pAVK im Stadium II nach Fontaine.

Arterielle Durchblutungsstörungen, wie periphere arterielle Verschlußkrankheit (pAVK), akuter cerebraler Insult, Augeninfarkt, Hörsturz, plazentare Durchblutungsstörungen etc., zählen zu häufig auftretenden Erkrankungen, die zum Teil äußerst langwierige Behandlungen (mit unterschiedlichem Erfolg) erfordern und eine erhebliche Beeinträchtigung darstellen.

Hydroxyethylstärke (HES) wurde bereits im Rahmen einer Hämodilutionstherapie bei Patienten mit arteriellen Durchblutungsstörungen eingesetzt. Therapeutisch wirksames Prinzip bei der Hämodilution ist die rheologische Verbesserung des Blutes, wie die Senkeng des Hämatokrits, die Reduktion der Plasmaviskosität sowie die Normalisierung der Erythrozytenaggregation. Beim Hämatokrit, bei der Plasmaviskosität und der Erythrozytenaggregation handelt es sich um rheologische Parameter, deren Größe Hinweise über die Fluidität des Blutes gibt (vgl. Kiesewetter, vasomed, 4. Jahrg., 10/92, S. 672-686 und EP-A 0402724).

In klinischen Studien wurde das Wirkprinzip bestätigt, wobei die klinische Relevanz einer verbesserter Rheologie beispielsweise bei peripherer arterieller Verschlußkrankheit (pAVK) in einem Anstieg der schumerzfreien Gehstrecke oder bei akutem Hirninfarkt in einer Verbesserung von Neuroscores gezeigt wurde.

In der Hämodilutionstherapie bei Durchblutungsstörungen zum Einsatz kommt derzeit handelsübliche mittelmolekulare Hydroxyethylstärke, wie HES 200/0,60 bis 0,66 und HES 200/0,5. Bei der Hämodilutionstherapie mit HES-Lösungen werden große Mengen dieser Lösungen infundiert, z.B. bis zu 10 l einer 10%igen HES-Lösung im Zeitraum von 10 Tagen bei akutem Hirninfarkt und bis zu 42 Tagen bei pAVK, was einer infundierten HES-Menge von 1 kg entspricht. Bei Untersuchungen wurde festgestellt, daß mehrfache Infusionen von HES-Lösungen zur Kumulation der Serum-HES-Konzentrationen führten. So z.B. wurden bei wiederholter Applikation von HES 450/0,7 (2 x wöchentlich 500 ml 6%ige HES-Lösung über einen Zeitraum von 6 Wochen) Serumkonzentrationen um 20 g/l erreicht und wurden 16 Wochen nach dieser wiederholten Applikation im Serum noch HES-Konzentrationen von etwa 1,32 g/l gefunden (Fortschritte der Medizin 97. Jahrgang, Nr. 40 (1979), S. 1809-1813).

Bei der Infusion der bekannten HES-Lösungen wurden weiterhin, bedingt durch die Kumulation der Serum-HES-Konzentrationen, Nebenwirkungen, wie allergische Reaktionen, z.B. Pruritus etc. beobachtet. Zur Vermeidung solcher Nebenwirkungen wurde von der Fachwelt bei der Therapie mit HES empfohlen, Patienten nicht mehr als 150 g Hydroxyethylstärke, entsprechend 3 x 500 ml HES 200/0,5 (10%ig) bzw. 5 x 500 ml HES 200/0,5 (6%ig) pro Woche zu infundieren (J. Koscielny, H. Kiesewetter et al, Hämodilution, Springer-Verlag, 1991, S. 214).

Für die Mikrozirkulation, deren Verbesserung ein wesentliches Prinzip bzw. das wesentlichste Prinzip in der Therapie der peripheren Durchblutungsstörungen ist, ist die Plasmaviskosität ein wichtiges Kriterium neben dem Hämatokrit und der Erythrozytenaggregation. Während bisher verwendete HES-Lösungen den Hämatokritwert absenken und die Erythrozytenaggregation verbessern, war ihr Einfluß auf die Senkung der Plasmaviskosität, um eine wesentliche Verbesserung der Mikrozirkulation zu bewirken, nicht ausreichend. Insofern wird zur Behandlung von peripheren arteriellen Verschlußkrankheiten HES oftmals mit vasoaktiven Substanzen kombiniert.

Im Hinblick auf diese Nachteile besteht daher nach wie vor ein Bedarf nach geeigneten Mitteln zur Verbesserung der Mikrozirkulation bei peripheren Durchblutungsstörungen, wie z.B. bei pAVK, die die Nachteile der bekannten Mittel nicht aufweisen.

Aufgabe der vorliegenden Erfindung ist es daher, ein Mittel zur Verbesserung der Mikrozirkulation bei peripheren Durchblutungsstörungen, insbesondere bei pAVK im Stadium II nach Fontaine bereitzustellen, mit denen die Mikrozirkulation wesentlich verbessert werden kann, und zwar auch ohne den Zusatz von vasoaktiven Substanzen.

Erfindungsgemäß wurde überraschend gefunden, daß mit einer HES-Spezifikation mit einem Molekulargewicht von M_{w} 110.000 bis 150.000, einem Substitutionsgrad MS von 0,38 bis 0,5, vorzugsweise von 0,38 bis 0,45, einem Substitutionsgrad DS von 0,32 bis 0,45, vorzugsweise von 0,32 bis 0,42, und einem Verhältnis von C₂/C₆ von 8 bis 20 eine signifikante Verbesserung der Plasmaviskosität erreicht wird und so eine Verbesserung der Mikrozirkulation bei peripheren Durchblutungsstörungen, insbesondere bei pAVK im Stadium II nach Fontaine, bewirkt werden kann.

Bedingt durch den Einsatz des natürlichen Ausgangsrohstoffes Amylopektin sowie durch das Herstellungsverfahren, bei dem im gewissen Umfang eine Spaltung der Polymerketten notwendig ist, liegt die Hydroxyethylstärke nicht als molekulareinheitliche Substanz mit definiertem Molekulargewicht vor, sondern als Gemisch von Molekülen unterschiedlicher Größe, die auch verschieden durch Hydroxyethylgruppen substituiert sind. Die Charakterisierung solcher Gemische bedarf der Zuhilfenahme statistisch gemittelter Größen. Zur Kennzeichnung des durchschnittlichen Molekulargewichtes dient daher das gemittelte Molekulargewicht M_{w}, wobei die allgemeine Definition dieses Mittelwertes in Krankenhauspharmazie (1987), S. 271 ff. angegeben ist.

Für die Erfassung der Substitution durch Hydroxyethylgruppen existieren zwei unterschiedlich definierte Substitutionsgrade. Der Substitutionsgrad MS (molar substitution) ist definiert als die durchschnittliche Anzahl von Hydroxyethylgruppen pro Anhydroglucoseeinheit. Es wird aus der Gesamtanzahl der Hydroxyethylgruppen in einer Probe, beispielsweise nach Morgan, durch Ätherspaltung und anschließender quantitativer Bestimmung von Ethyljodid und Ethylen, die hierbei gebildet werden, ermittelt. Der Substitutionsgrad DS (degree of substitution) ist als der Anteil der substituierten Anhydroglucoseeinheiten aller Anhydroglucoseeinheiten definiert. Er kann aus der gemessenen Menge der unsubstituierten Glucose nach Hydrolyse einer Probe bestimmt werden.

Das Verhältnis der Substitution von C₂/C₆ bedeutet das Verhältnis der Zahl der in der 2-Stellung substituierten Anhydroglucoseeinheiten zu der Zahl der in 6-Stellung substituierten Anhydroglucoseeinheiten der Hydroxyethylstärke.

Die Infusion der erfindungsgemäß verwendeten HES-Spezifikation führt zu einer signifikanten Verringerung der Plasmaviskosität bereits unmittelbar nach Infusionsende, zu einer signifikanten Absenkung des Hämatokrits sowie zu einer signifikanten Verringerung der Erythrozytenaggregation.

Durch diese rheologischen Veränderungen des Blutes wird eine signifikante Verbesserung der Mikrozirkülation bei peripheren Durchblutungsstörungen, wie z.B. bei pAVK im Stadium II nach Fontaine erzielt. Die rheologischen Eigenschaften des Blutes werden durch die erfindungsgemäß verwendete HES-Spezifikation überraschend und in einem stärkeren Maße verändert, als dies durch die bisher für die Hämodilutionstherapie eingesetzten Hydroxyethylstärken möglich ist. So wird die Plasmaviskosität durch die erfindungsgemäß angewandte HES-Spezifikation gegenüber der Hydroxyethylstärke 200/0,5 und der Hydroxyethylstärke 500/0,3 erheblich verändert. Nach Infusion der erfindungsgemäß verwendeten HES-Spezifikation fiel die Plasmaviskosität gegen Untersuchungsende hin ab, während unter vergleichbaren Bedingungen nach Infusion von HES 200/0,5 und HES 500/0,3 die Plasmaviskosität gegen Untersuchungsende deutlich anstieg. Im Vergleich zu HES 200/0,5, der in der Medizin für die Hämodilutionstherapie meist eingesetzten Hydroxyethylstärke, fiel bei der erfindungsgemäß angewandten HES-Spezifikation die Plasmaviskosität bereits gegen Infusionsende deutlich ab und war bei der erfindungsgemäß angewandten HES-Spezifikation die Plasmaviskosität im zeitlichen Verlauf signifikant erniedrigt.

Auch nach Mehrfachdosierung mit der erfindungsgemäß angewandten HES-Spezifikation wurde die Plasmaviskosität positiv beeinflußt.

Weiterhin wurde überraschend gefunden, daß die Serumelimination der erfindungsgemäß verwendeten HES-Spezifikation nach einmaliger Infusion sehr gut ist. Insbesondere war nach mehrfacher Infusion der erfindungsgemäß angewandten HES-Spezifikation keine Kumulation (weniger als 1 mg/ml) von HES-Konzentrationen im Serum zu beobachten. In der verbleibenden Serumrestfraktion an HES lag das mittlere Molekulargewicht M_{w} bei 70 kda. Dies entspricht der Nierenschwelle für die Ausscheidung von Hydroxyethylstärke.

Die erfindungsgemäß verwendete HES-Spezifikation wird in üblicher Weise hergestellt, wie z.B. in der DE-A 39 19 729 beschrieben.

Die Verabreichung der erfindungsgemäß verwendeten HES erfolgt in Form von wässrigen Lösungen, in denen die HES in üblicherweise verwendeten Konzentrationen vorliegt. Geeignet sind Konzentrationen im Bereich von 5 bis 15 Gew.-%, vorzugsweise 6 bis 10 Gew.-%. Geeignete Konzentrationen sind die beispielsweise 6% Gew.-%ige Konzentration und die 10 Gew.-%ige Konzentration, wobei die 6 Gew.-%ige Konzentration bevorzugt ist. Diese Lösungen können übliche Hilfs- und Zusatzstoffe, enthalten.

Die nach Infusion der erfindungsgemäß angewandten HES-Spezifikation bewirkten rheologischen Veränderungen des Blutes bei schneller Serumelimination ohne Kumulationsgefahr von hochmolekularen Serum-HES-Bestfraktionen, auch bei mehrfacher Anwendung, gewährleisten eine wirksame und sichere Anwendung der erfindungsgemäß eingesetzten HES-Spezifikation über einen längeren Zeitraum bei Patienten mit arteriellen Durchblutungsstörungen, insbesondere bei pAVK im Stadium II nach Fontaine.

Die nachfolgenden Beispiele dienen der Erläuterung der vorliegenden Erfindung.

### Beispiel 1

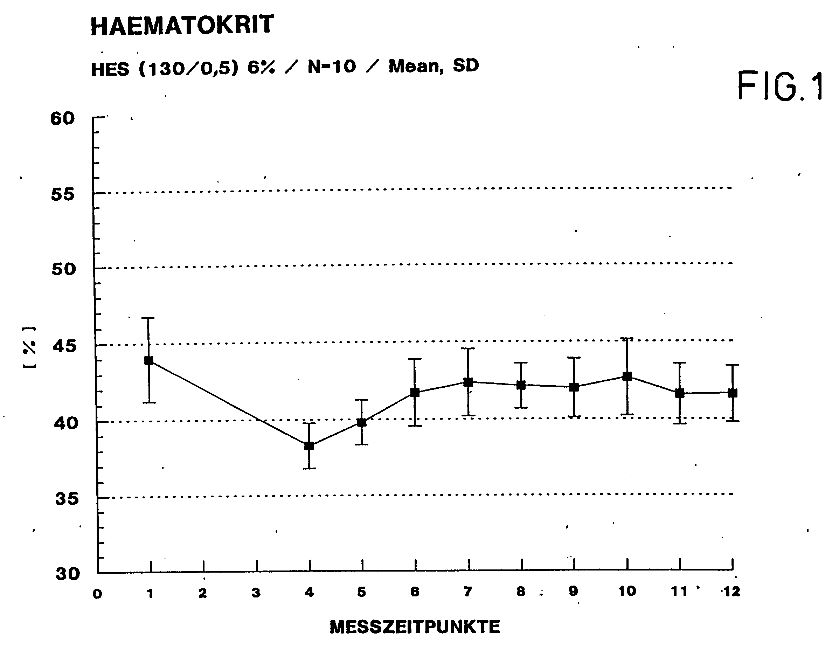

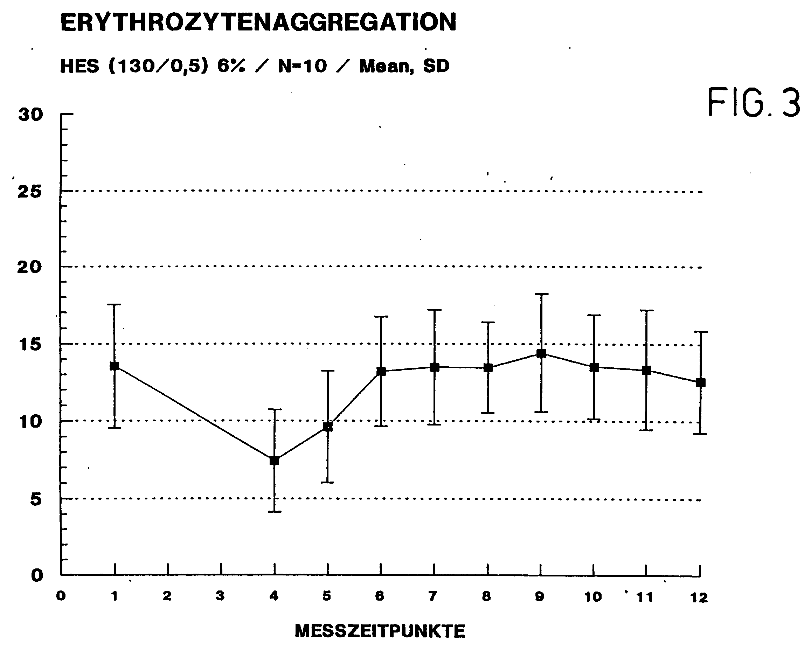

In einer Studie wurde 10 gesunden männlichen Probanden einmalig 500 ml einer 6 Gew.-%igen Lösung von HES 130/0,5, d.h. HES mit M_{w} von 136.800, MS von 0,396, DS von 0,346 und einem Verhältnis von C₂/C₆ von 10,8, innerhalb von 30 Minuten i.v. infundiert. Der Untersuchungszeitraum betrug 72 Stunden. Innerhalb dieses Zeitraumes wurden der Hämatokrit, die Plasmaviskosität und die Erythrozytenaggregation des Blutes zu bestimmten Meßzeitpunkten bestimmt. Die Untersuchungsergebnisse sind aus den Figuren 1 bis 3 ersichtlich.
Figur 1 zeigt den Verlauf des Hämatokrits im Untersuchungszeitraum. Der Hämatokrit nahm zum Infusionsende (Meßzeitpunkt 4) um 15% ab und blieb auch 3 Tage nach der Infusion noch 6% unter dem Ausgangswert. Der p-Wert im zeitlichen Verlauf ist p <0,001.
Figur 2 zeigt den Verlauf der Plasmaviskosität. Bereits unmittelbar nach Infusionsende (Meßzeitpunkt 4) war die Plasmaviskosität um 4% erniedrigt und nach einer Stunde (Meßzeitpunkt 5) wieder auf ihrem Ausgangsniveau. Der p-Wert im zeitlichen Verlauf ist p <0,05.
Figur 3 zeigt den Verlauf der Erythrozytenaggregation. Die Erythrozytenaggregation war bei Infusionsende (Meßzeitpunkt 4) fast halbiert, erreichte jedoch nach 2 Stunden wieder ihr Ausgangsniveau. Der p-Wert im zeitlichen Verlauf ist p <0,001.

Die gefundenen Werte zeigen eine ausgeprägte hämatokritsenkende sowie stark ausgeprägte erythrozytenaggregationssenkende Wirkung. Besonders auffällig ist die durch die erfindungsgemäß verwendete HES 130/0,5-Lösung bewirkte Senkung der Plasmaviskosität bereits nach Infusionsende.

### Beispiel 2

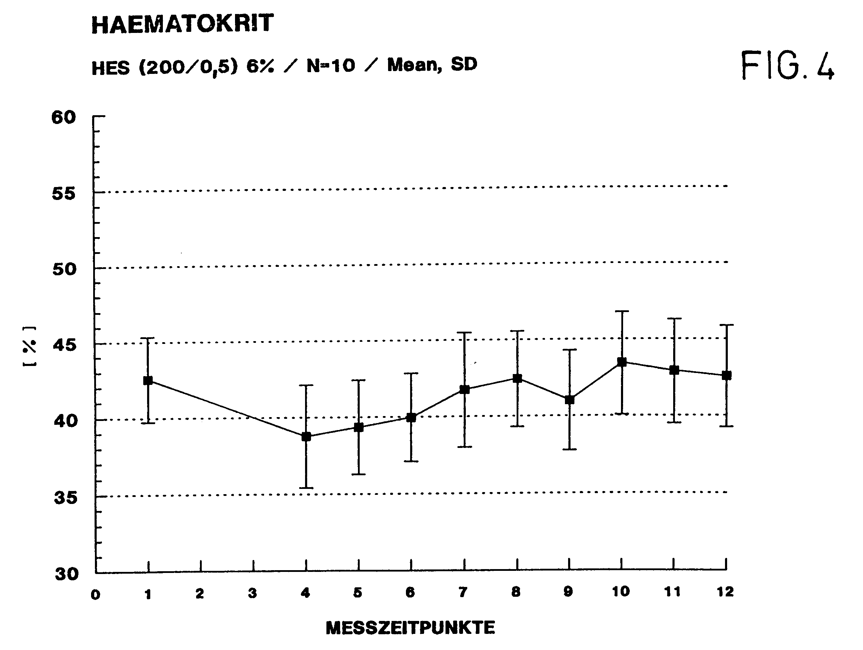

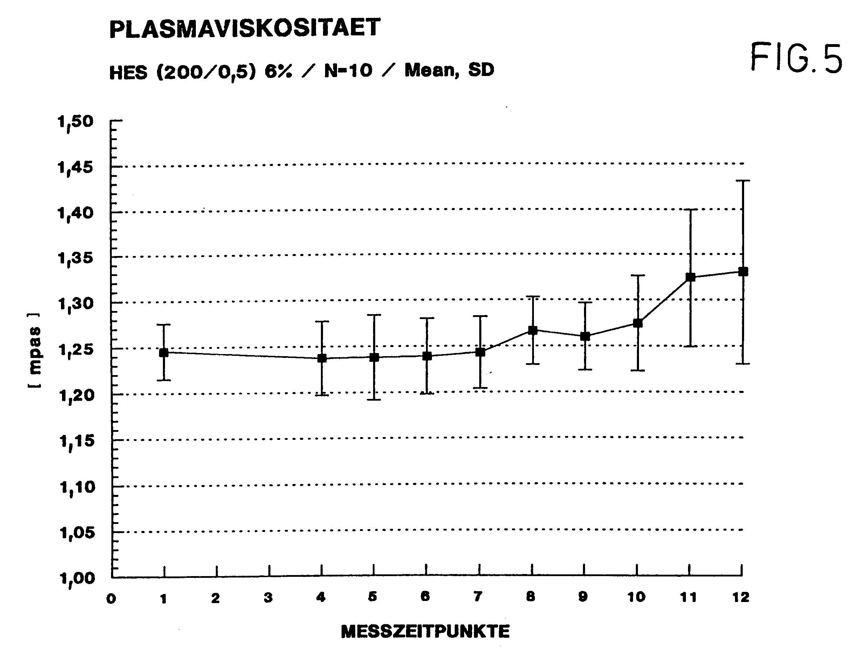

Zu Vergleichszwecken wurde unter den gleichen Bedingungen, wie sie vorstehend in Beispiel 1 beschrieben wurden, 10 gesunden männlichen Probanden einmal 500 ml einer 6 Gew.-%igen Lösung von HES 200/0,5 innerhalb von 30 Minuten i.v. infundiert. Der Untersuchungszeitraum betrug 72 Stunden. In diesem Zeitraum wurden der Hämatokrit und die Plasmaviskosität zu bestimmten Meßzeitpunkten, die mit den Meßzeitpunkten gemäß Beispiel 1 übereinstimmten, bestimmt. Die erhaltenen Ergebnisse sind aus den Figuren 4 und 5 ersichtlich.

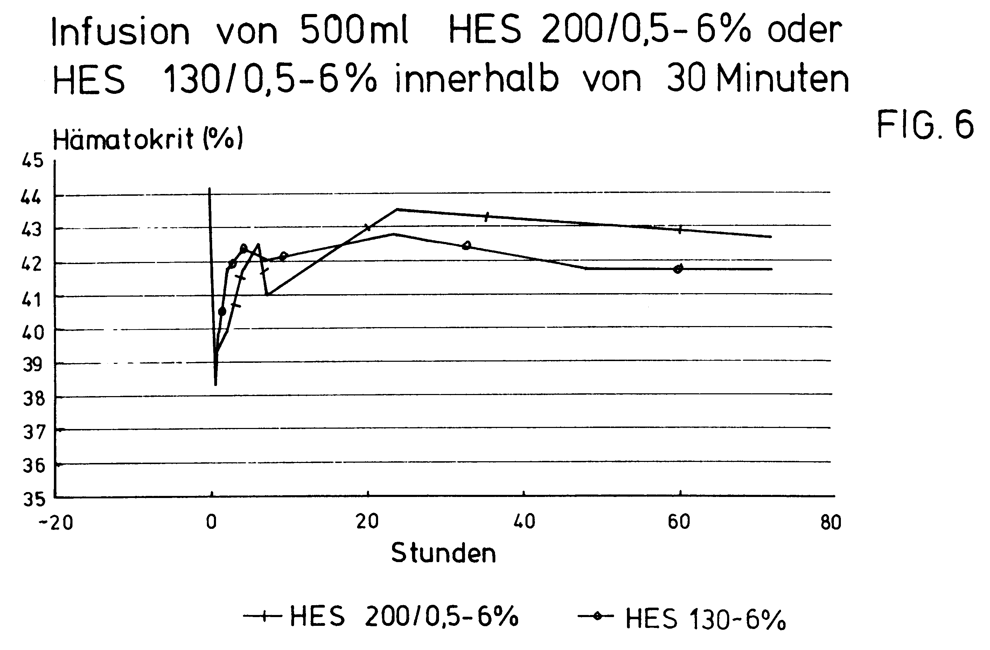

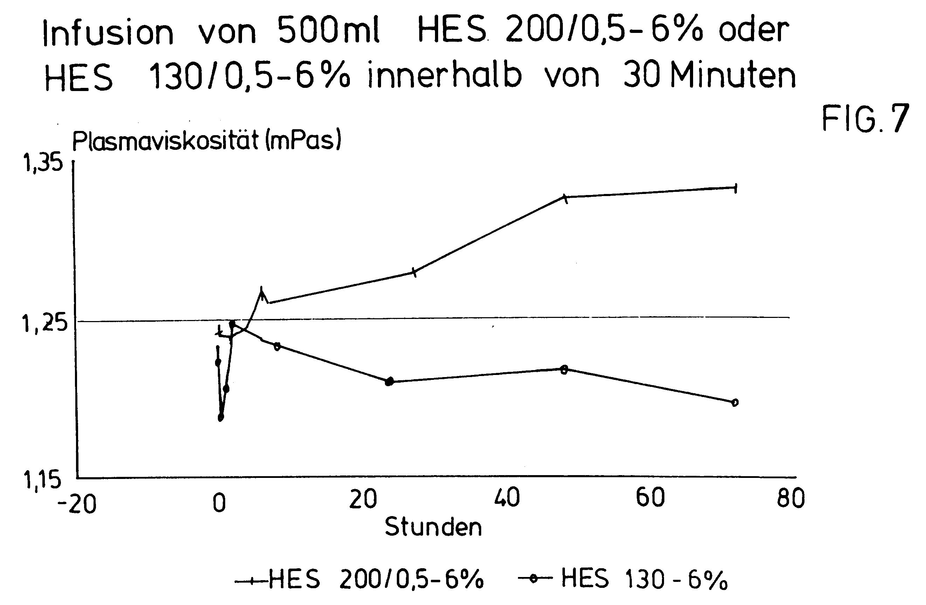

Die Untersuchungsergebnisse, wie sie vorstehend in den Beispielen 1 und 2 erhalten wurden, wurden zum Zwecke des besseren Vergleiches in den Figuren 6 und 7 gegenübergestellt. Während die Absenkung des Hämatokrits (vgl. Figur 6) bei HES 130/0,5 und HES 200/0,5 etwa einen vergleichbaren Verlauf aufweist, ist die Plasmaviskosität (vgl. Figur 7) der HES 130/0,5 im Vergleich zur HES 200/0,5 im zeitlichen Verlauf signifikant erniedrigt.

### Beispiel 3

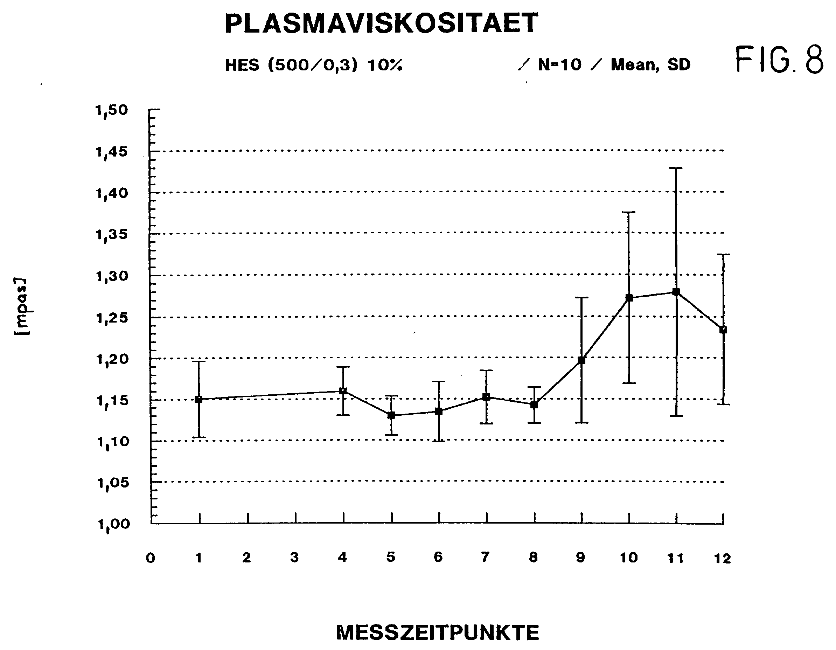

Zu Vergleichszwecken wurden die in Beispiel 1 beschriebenen Untersuchungen unter den gleichen Bedingungen wiederholt mit der Ausnahme, daß anstelle der 6 Gew.-%igen Lösung von HES 130/0,5 eine 10 Gew.-%ige Lösung von HES 500/0,3 (M_{w} 500.000, MS 0,28 und ein Verhaltnis von C₂/C₆ von 8,7) infundiert wurde. Im Untersuchungzeitraum von 72 Stunden wurde die Änderung der Plasmaviskosität zu bestimmten Meßzeitpunkten untersucht. Die bei diesen Untersuchungen erhaltenen Ergebnisse sind aus Figur 8 ersichtlich. In der nachfolgenden Tabelle sind die Änderungen der Plasmaviskosität zusammengestellt.

| PLASMAVISKOSITÄT HES (500/0,3) 10 % | | | | | |
|---|---|---|---|---|---|
| Param. MZP * | Mean | SD | Min | Max | N |
| Plasmaviskos. 1 | 1,151 | 0,046 | 1,050 | 1,205 | 10 |
| Plasmaviskos. 4 | 1,159 | 0,030 | 1,110 | 1,205 | 10 |
| Plasmaviskos. 5 | 1,130 | 0,024 | 1,095 | 1,165 | 10 |
| Plasmaviskos. 6 | 1,134 | 0,036 | 1,063 | 1,170 | 10 |
| Plasmaviskos. 7 | 1,152 | 0,032 | 1,100 | 1,190 | 10 |
| Plasmaviskos. 8 | 1,142 | 0,022 | 1,105 | 1,187 | 10 |
| Plasmaviskos. 9 | 1,197 | 0,076 | 1,130 | 1,390 | 10 |
| Plasmaviskos. 10 | 1,272 | 0,103 | 1,175 | 1,507 | 10 |
| Plasmaviskos. 11 | 1,279 | 0,150 | 1,120 | 1,645 | 10 |
| Plasmaviskos. 12 | 1,234 | 0,090 | 1,135 | 1,400 | 10 |

| | | | | | |
|---|---|---|---|---|---|
| * Param. MZP : Parameter - Name und Meßzeitpunkt Test nach FREIDMANN über zeitlichen Verlauf *** [n.s.: p > 0.05 * : p < = 0.05 ** : p < = 0.01 *** : p < = 0.001] | | | | | |

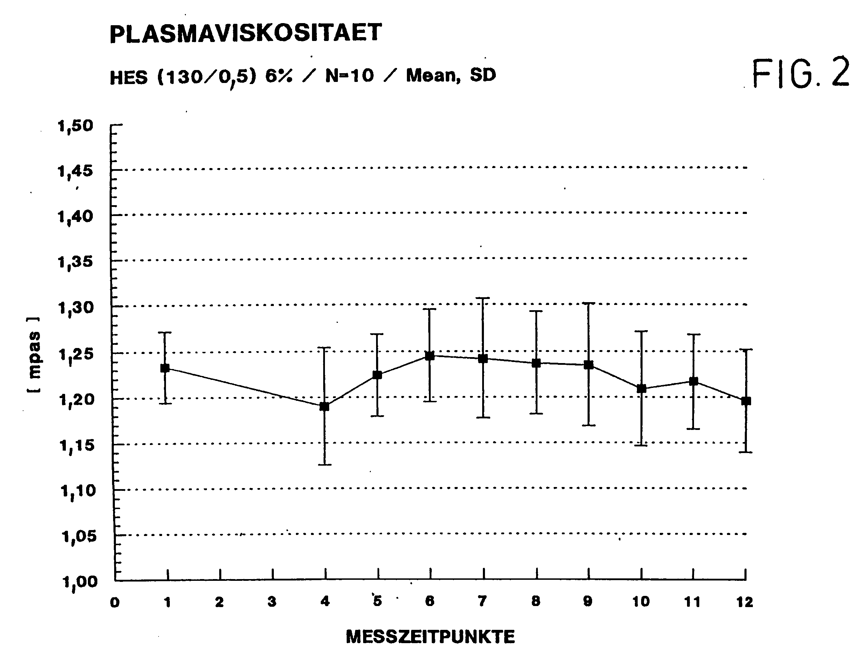

Ein Vergleich der Ergebnisse mit dem Verlauf der Änderung der Plasmaviskosität von HES 130/0,5 (vgl. Figur 2) zeigt einen unterschiedlichen Verlauf. Auch mit HES 500/0,3, ebenso wie bei HES 200/0,5, steigt gegen Ende des Untersuchungszeitraumes die Plasmaviskosität unvorteilhaft an.

### Beispiel 4

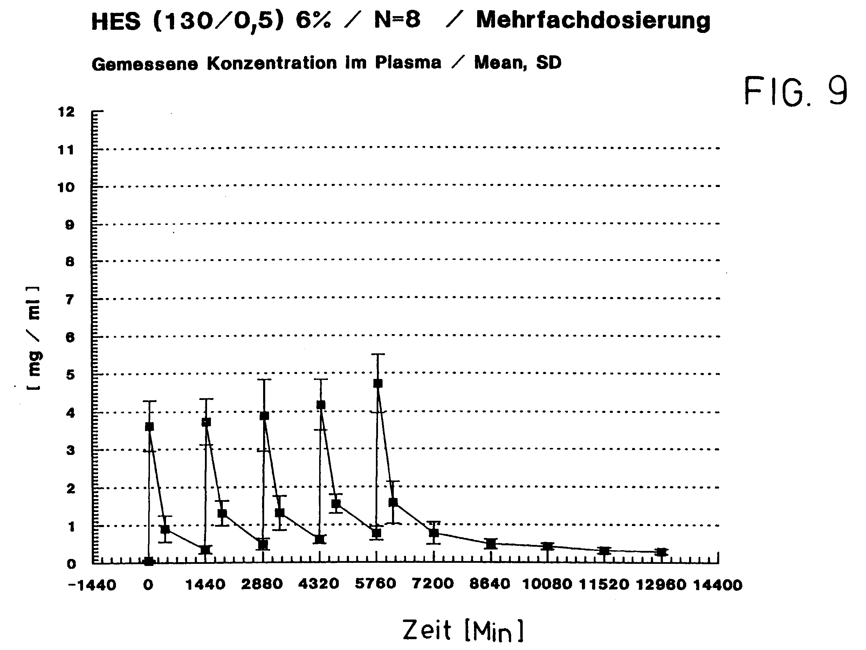

In einer weiteren Studie wurde 8 gesunden männlichen Probanden an 5 aufeinanderfolgenden Tagen jeweils 500 ml einer 6 Gew.-%igen Lösung von HES 130/0,5 innerhalb von 30 Minuten infundiert. Über den Untersuchungszeitraum von 10 Tagen wurde die Serum-HES-Konzentration bei bestimmten Meßpunkten bestimmt. Die erhaltenen Werte sind aus der Figur 9 ersichtlich.

Figur 9 zeigt den Verlauf der Serum-HES-Konzentrationen, der ein typisches "Sägezahnprofil" aufweist. Bei Infusionsende wurden die höchsten Konzentrationen gemessen, nach 6,5 weiteren Stunden (Bereich bis 1,6 mg/ml) sowie am jeweils folgenden Morgen waren die Konzentrationen bereits deutlich abgefallen (Bereich <0,8 mg/ml). Die höchste gemessene Konzentration betrug nach der fünften Infusion 4,73 mg/ml, jedoch bereits am Morgen des 6. Tages sowie an den folgenden Tagen war die Konzentration <1,0 mg/ml. Ein statistisches Modell, das aufgrund der vorliegenden Daten eine tägliche Dauerinfusion simulierte, berechnete eine Höchstkonzentration nach Infusionsende von 5,74 mg/ml.

Die mittleren Molekulargewichte M_{w} der Serum-HES-Restfraktionen, gemessen 7 Stunden nach Infusionsbeginn bzw. an den Tagen 6 bis 10, lagen im Bereich von 70 bis 80 kda, d.h. im Bereich der Nierenschwelle für Hydroxyethylstärke.

## Patentansprüche

1. Verwendung einer Hydroxyethylstärke mit einem Molekulargewicht M_{w} von 110.000 bis 150.000, einem Substitutionsgrad MS von 0,38 bis 0,5, einem Substitutionsgrad DS von 0,32 bis 0,45 und einem Verhältnis von C₂/C₆ von 8 bis 20 bei der Herstellung eines Arzneimittels zur Verbesserung der Mikrozirkulation bei peripherer arterieller Durchblutungsstörung.

2. Verwendung der Hydroxyethylstärke nach Anspruch 1 mit einem Molekulargewicht M_{w} von 110.000 bis 150.000, einem Substitutionsgrad MS von 0,38 bis 0,45, einem Substitutionsgrad DS von 0,32 bis 0,42 und einem Verhältnis von C₂/C₆ von 8 bis 20.

3. Verwendung der Hydroxyethylstärke nach Anspruch 1 oder 2 bei der Herstellung eines Arzneimittels zur Verbesserung der Mikrozirkulation bei pAVK im Stadium II nach Fontaine.

4. Verwendung der Hydroxyethylstärke nach Anspruch 1,2 oder 3 bei der Herstellung eines Arzneimittels in einer 6 oder 10 Gew.-%igen Lösung.

## Claims

1. Use of a hydroxy ethyl starch with a molecular weight M_{w} of 110,000 to 150,000, a substitution degree MS of 0.38 to 0.5, a substitution degree DS of 0.32 to 0.45 and a ratio of C₂ : C₆ of 8 to 20 in the manufacture of a medicament for improving microcirculation in cases of peripheral arterial blood flow disturbance,

2. Use of the hydroxy ethyl starch according to claim 1 with a molecular weight M_{w} of 110,000 to 150,000, a substitution degree MS of 0.38 to 0.45, a substitution degree DS of 0.32 to 0.42 and a ratio of C₂: C₆ of 8 to 20.

3. Use of the hydroxy ethyl starch according to claim 1 or 2 in the manufacture of a medicament for improving microcirculation in cases of Stage II (Fontaine) peripheral arterial occlusion disease.

4. Use of the hydroxy ethyl starch according to claim 1, 2 or 3 in the manufacture of a medicament in a 6% by weight or 10% by weight solution.

## Revendications

1. Utilisation d'un hydroxyéthylamidon possédant un poids moléculaire M_{w} de 110.000 à 150.000, un degré de substitution MS de 0,38 à 0,5, un degré de substitution DS de 0,32 à 0,45 et un rapport C₂/C₆ de 8 à 20 lors de la préparation d'un médicament pour améliorer la microcirculation en cas de troubles de l'irrigation sanguine artérielle périphérique.

2. Utilisation de l'hydroxyéthylamidon selon la revendication 1, possédant un poids moléculaire M_{w} de 110.000 à 150.000, un degré de substitution MS de 0,38 à 0,45, un degré de substitution DS de 0,32 à 0,42 et un rapport C₂/C₆ de 8 à 20.

3. Utilisation de l'hydroxyéthylamidon selon la revendication 1 ou 2 dans la préparation d'un médicament pour améliorer la microcirculation dans la pAVK du stade II selon Fontaine.

4. Utilisation de l'hydroxyéthylamidon selon la revendication 1, 2 ou 3 lors de la préparation d'un médicament dans une solution dont la concentration s'élève à 6 ou à 10% en poids.
